# EUROPEAN PATENT APPLICATION

(11) **EP 2 424 269 A2**
(43) Date of publication of application: **29.02.2012**
(21) Application number: 11003155.6
(22) Date of filing: 14.04.2011
(51) Int. Cl.: H04R 1/10

(54) **Earphone having sound insulation means**

(30) Priority: 30.08.2010 JP 2010206088
(71) Applicant: Kakumoto, Jun-ichi, Tokushima-ken (JP)
(72) Inventor: Kakumoto, Jun-ichi, Tokushima-ken (JP)
(74) Representative: TER MEER - STEINMEISTER & PARTNER GbR

(57) **Abstract**

The present invention relates to an inexpensive environmental noise reduction type inner earphone which allows a user to listen music with high quality of sound and with a small volume under noisy environments making noise of 86dB or more such as in aircraft seats, subways, trains and so on, and particularly which is capable of blocking a high level of environmental noise and regeneration sound passing through the inner earphone by filling a space formed between an ear and the inner earphone with a sound insulation element made of porous soft plastic. The earphone includes a frequency characteristic correction circuit which serves to correct a sound insulation characteristic of the sound insulation element between a voice coil of the earphone and a plug of the earphone and includes a resistor and a capacitor.

## Description

### CROSS-REFERENCE TO RELATED APPLICATION

This application claims the benefit of Japanese Application No. 2010-206088, filed on August 30, 2010, with the Japan Patent Office, the disclosure of which is incorporated herein by reference.

### BACKGROUND OF THE INVENTION

### Field of the Invention

The present invention relates to an earphone, and more particularly, to an earphone having sound insulation means for blocking a portion of a space formed between an earphone body and an external auditory canal. The present invention also relates to a sound insulation material, a sound absorption material, an environmental noise reduction, an earphone manufacturing technology and an electronic circuit filter.

### Description of the Related Art

In recent years, earphones or players having an environmental noise reduction capability have been being sold. Such devices may be generally classified into an active type and a passive type. The present invention involves a passive type earphone.

Throughout the specification including the claims, technical terms indicated by { · } take precedence over other technical terms. Technical terms defined in the claims are equally applied to the specification.

As a background art for passive type earphones, there are two important factors, i.e., a method of enhancing an external noise blocking performance of an earphone while maintaining an earphone regeneration performance of the earphone in order to block a sound propagation path ranging from an environmental noise source to an auditory organ, and improvement of material and shape of sound insulation elements for enhancement of sound insulation performance of {gap between an ear and an earphone}.

For this purpose, Japanese Patent Application Publication No. H08-275298 discloses an earplug-typed earphone mounting mechanism and an earphone and Japanese Patent Application Publication No. H10-511832 discloses an ear cushion-attached headset and a cushion compression limiting device. However, in some case, since environmental noises propagate through an earphone structure to reach an auditory organ, the techniques disclosed in these documents cannot provide a high level of satisfaction of a noise reduction performance.

In addition, Japanese Patent Application Publication No. 2002-200109 discloses an earplug-typed electroacoustic transducer, which reduces noises by directly delivering {vibration of a diaphragm of an earphone} to a {sound insulation element as the earplug}. However, with this transducer disclosed in the Patent document, it is difficult to obtain high quality of sound performance since a sound insulation material is inherently incompatible to {direct propagation of sound using a mechanical vibration from a material having different propagation characteristics}.

Conventional sound insulation elements cannot have sufficient sound insulation effects under noisy environments such as {in subways, trains, engines and wind of aircrafts, etc.} making environmental noise of 87dB or more.

### SUMMARY OF THE INVENTION

Accordingly, it is an object of the present invention to provide an inexpensive environmental noise reduction type inner earphone which allows a user to listen music with high quality of sound and with a small volume even under noisy environments making environmental noise of 86dB or more such as in aircraft seats, subways, trains and so on.

It is another object of the present invention to provide noise reduction which satisfies conditions of {inexpensiveness, easy manufacture, convenient use, high reduction performance and high quality of regeneration sound} and which allows a user to listen music and call sound with high quality without increasing a volume under noisy environments making environmental noise of 87dB or more.

To achieve the above objects, according to an aspect of the invention, there is provided an earphone including an earphone body which includes a voice coil and outputs a regeneration sound through radiating holes; a sound insulation element which is made of deformable sound insulating porous plastic, covers the radiating holes of the earphone body, and fills a space formed between the earphone body and an entrance of an external auditory canal; and a hollow tube which is mounted on the radiating holes of the earphone body and forms an aggregate such that the sound insulation element is combined to the earphone body.

Preferably, the hollow tube includes an engagement projection formed on the circumference of the hollow tube and by which the sound insulation element is fixed to the hollow tube.

Preferably, the earphone includes a high sound range attenuation correction circuit including a capacitor and a resistor connected in parallel between the voice coil of the earphone and a plug of the earphone.

The present invention can be applied to a wide range of goods since it is {simple, highly efficient and inexpensive} and provides {regeneration environments having environmental noise reduction}.

In addition, the present invention can provide more stable regeneration environments against apprehensive auditory disturbance since a small volume is delivery to an eardrum of a user and the user can listen music with {high quality of sound}.

In addition, since the earphone of the present invention is inexpensive, a majority of persons can comfortably enjoy with {moderate volume and high quality of sound} for a long time in {trains, aircrafts and so on}.

In addition, {mobile phone call conditions} are highly improved under noisy environments, which can serve to reduce loud call {as may be often the case under noisy environments} as well as convenient use.

On the other hand, although the attenuation characteristic of the earphone of the present invention requires appropriate adjustment, since the attenuation characteristics can be simply adjusted according to a margin of output of a player which is likely to be used under highly noisy environments, compensation of attenuation in a middle and low sound range can be covered by increasing a playback volume.

For example, a {volume of seat entertainment in an aircraft} is designed to be {sufficiently heard under noisy environments}. Therefore, an appropriate volume can be secured when such noise is blocked along with a playback signal.

In reality, results of experiments in an aircraft showed that the earphone of the present invention is sufficiently used with a range of from a conventional level to a small volume of 10dB to 20dB although an {earphone equipped within the aircraft} has to significantly increase a volume for listening.

Accordingly, it is possible to greatly reduce an auditory fatigue due to a large volume even when the earphone is used for a long time. Further, the earphone of the present invention can be employed for conventional players, which may result in lowering the barrier to market entry.

On the other hand, the earphone of the present invention has substantially the same effect of sound leakage as conventional.

### BRIEF DESCRIPTION OF THE DRAWINGS

FIG. 1 is an explanatory view for an embodiment of the present invention;
FIG. 2 is an explanatory view for use condition of the embodiment of FIG. 1;
FIG. 3 is an explanatory view for a conventional technique;
FIG. 4 is an explanatory view for an embodiment of the present invention;
FIG. 5 is an explanatory view for a sound insulation characteristic of a conventional technique;
FIG. 6 is an explanatory view for a sound insulation characteristic of an embodiment of the present invention;
FIG. 7 is an explanatory view for a regeneration characteristic of an embodiment of the present invention;
FIG. 8 is an explanatory view for a regeneration characteristic of an embodiment of the present invention;
FIG. 9 is an explanatory view for a regeneration characteristic of an embodiment of the present invention; and
FIG. 10 is a CR type high sound range attenuation compensation circuit diagram of an embodiment of the present invention.

### DETAILED DESCRIPTION OF THE PREFERRED EMBODIMENT

In general, in order to enhance an earphone regeneration performance, it is preferable that {rear side of a diaphragm} is outwardly opened. However, it is preferable that an earphone of a conventional type of {blocking environmental noise} has a {structure where a rear side of a diaphragm of an earphone is sealed} in either {active type or passive type} for {the purpose of blocking environmental noise}. Although the rear side of the earphone may be simply sealed, a generation performance may be affected by such a sealing.

In particular, there is a product subjected to delicate workmanship such as installing a duct from a rear side of a diaphragm and opening an ear side opposing the duct in order to avoid {noticeable deterioration due to sealing} of a low sound regeneration performance. In brief, {enhancement of sound performance of earphone itself} cannot be substantially compatible with {enhancement of low sound regeneration performance of earphone}.

Accordingly, in the present invention, by filling a space formed between an ear and an inner earphone with a sound insulation element made of porous soft plastic, a regeneration sound is blocked along with a high level of environmental noise passing the inner earphone. In this case, according to an embodiment of the present invention, a frequency characteristic correction circuit, which serves to correct a sound insulation characteristic of the sound insulation element and is composed of resistors and capacitors, is interposed between a voice coil and a plug of the earphone.

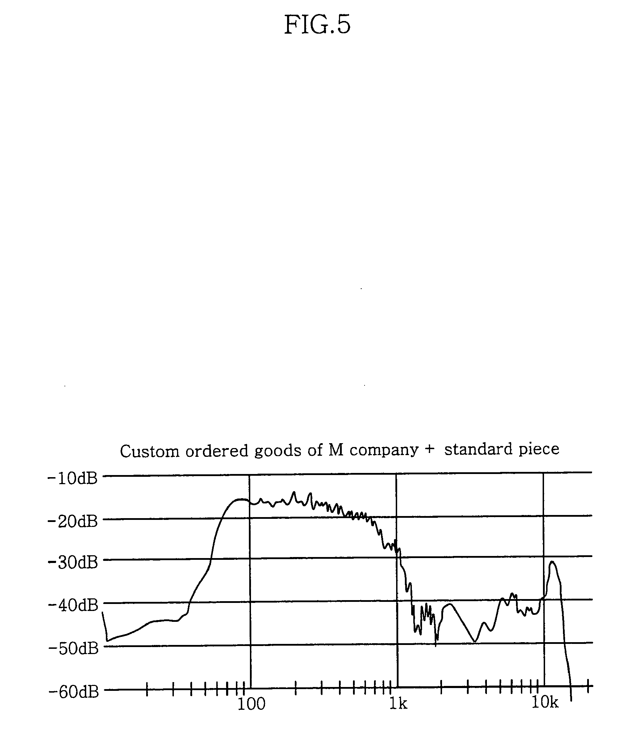

FIGs. 5 to 9 are views showing measured characteristics, where a horizontal axis represents a frequency and a vertical axis represents a {{relative voltage measured through an amplifier} of a measurement microphone output}. All of the figures having the same reference level show {measurement data in sufficient measurement environments} {in comparing respective characteristics}.

In the present invention, by filling a {space formed between the inner earphone and an entrance of an external ear (a portion of an external auditory canal)} with a sound insulating material, both of {environmental noise to be reduced} and {music or announcement signal to be listened} are attenuated. This method performs {sound insulation using a material having a high performance sound insulation effect for regeneration sound}, which results in attenuation of volume of regeneration sound, particularly high sound.

Sound quality can be corrected by correcting a dependency of attenuation on frequency. {Noise reduction type earphone with high convenience} can be realized by {providing this correction for an earphone regeneration characteristic} or {inserting an {electronic or electrical circuit} in an earphone}.

As a result, sound insulation performance of environmental noise can be enhanced and {attenuation of regeneration sound} is corrected to achieve normal sound quality and improve a S/N ratio.

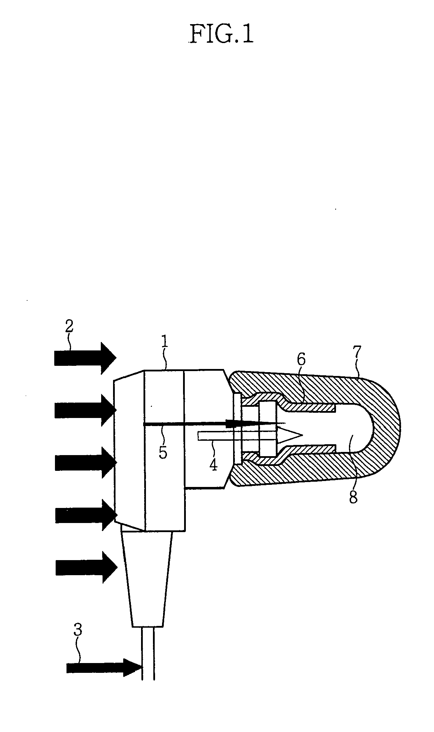

FIG. 1 is a view showing one embodiment of the present invention.

Reference numeral 1 denotes an earphone body, reference numeral 2 denotes environmental noise, reference numeral 3 denotes friction noise, reference numeral 4 denotes an earphone regeneration signal, reference numeral 5 denotes a {combination of environmental noise and friction noise} which reaches an auditory sense via the earphone body, reference numeral 6 denotes a hollow tube which {facilitates mounting of a soft sound element, which will be described later, on the earphone body and promotes stability of sound insulation performance of a sound insulation material (in use)}, reference numeral 7 denotes a sound insulation element which is made of {deformable sound insulation porous plastic} for {blocking a portion of a space which is formed between the earphone body and an external auditory canal}, and reference numeral 8 denotes a space which is formed in an inner side of the sound insulation element.

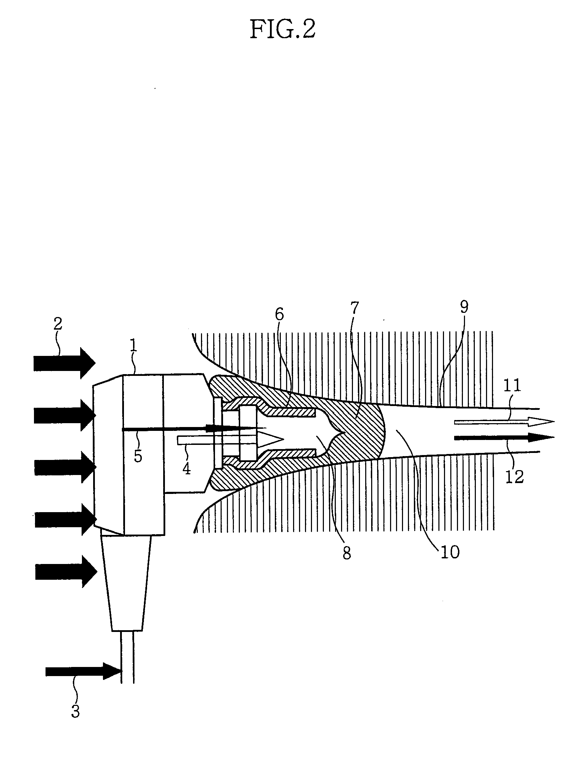

FIG. 2 is a view showing a state where the embodiment of FIG. 1 is mounted on an ear.

In FIG. 2, reference numerals 1 to 8 denote the same elements as FIG. 1. Reference numeral 9 denotes a contour of the external auditory canal, reference numeral 10 denotes the external auditory canal, reference numeral 11 denotes an earphone regeneration signal which reaches the auditory sense via the sound insulation element, and reference numeral 12 denotes environmental noise which reaches the auditory sense via the sound insulation element.

The {earphone regeneration sound} 4 and the {environmental noise and friction noise which enter via the earphone} 5 reach the auditory sense via the external auditory canal while being attenuated by a sound insulation effect of the sound insulation element 7. As apparent from the figure, the environmental noise and the friction noise reaching the auditory sense are greatly attenuated by the {sound insulation effect of the sound insulation element} as a portion of the external auditory canal is blocked by the sound insulation element. Although the earphone regeneration sound is naturally attenuated likewise, by applying a {reverse characteristic of an attenuation effect by the sound insulation element} to the {earphone regeneration sound} in advance using a {high sound range attenuation compensation electronic circuit or high sound range attenuation compensation type signal processing}, the {attenuation by the sound insulation element} can be corrected. As a result, the original regeneration sound quality can be recovered to {substantially the same {sound quality} as a state where no sound insulation element is installed}, which results in reduction of the {environmental noise and a friction sound of a cord} by only {a degree of sound insulation effect of the sound insulation element}.

At this time, the hollow tube 6 serves to {secure stable sound insulation performance} under a {state where the sound insulation element is mounted on the ear} by promoting stability of a {shape of a sound exit of the earphone}, that is, by {securing the space 8 stably}. Many experiments showed that {quality of material of the sound insulation element}, {volume of a portion of the sound insulation element blocking the external auditory canal} and {shape of the space 8 in the inner side of the sound insulation element} had a great effect on the sound insulation characteristic under a state where the earphone is inserted in the ear, the role of the hollow tube is important to obtain a stable sound insulation characteristic.

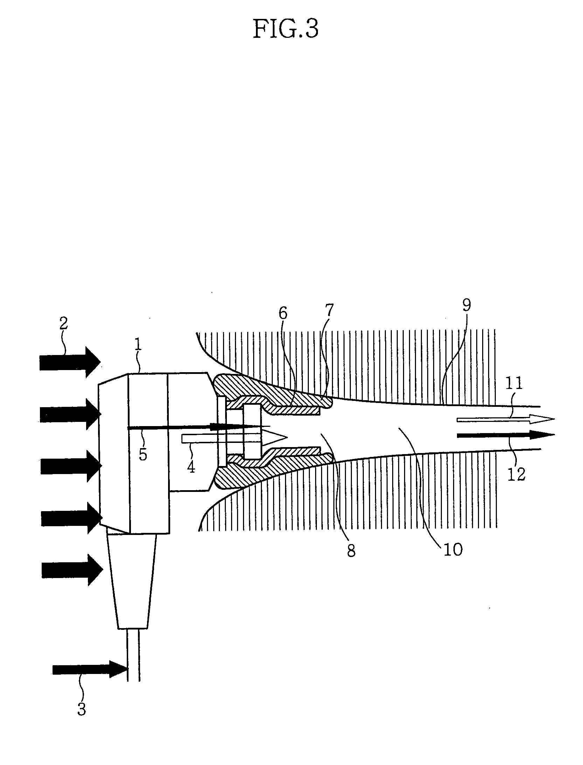

FIG. 3 is a view showing a state where a conventional earphone is inserted in an ear.

An earphone regeneration signal reaches an auditory sense via an external auditory canal without being blocked. Naturally, since {environmental noise and friction noise of a cord} reach the auditory sense via the earphone without being shielded, sound insulation performance of {environmental noise and friction noise} of the earphone itself has to be high.

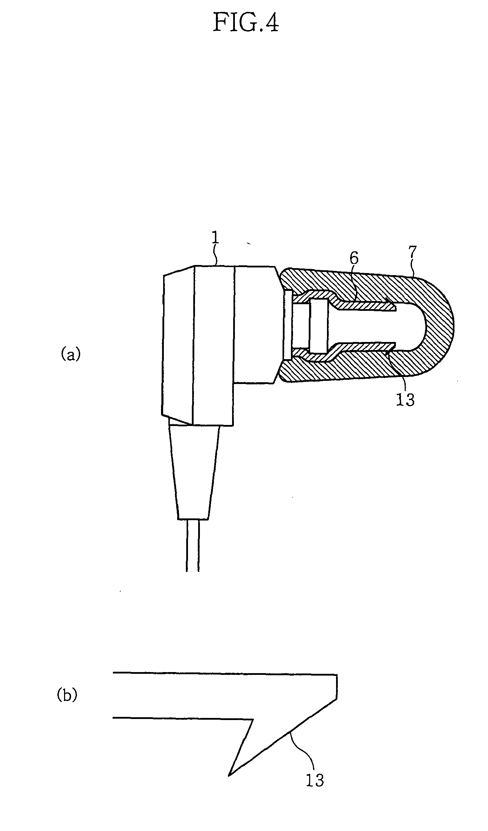

FIG. 4 shows one embodiment of a composite sound insulation element of the present invention.

In the figure, the same reference numerals as FIG. 1 have the same functions as FIG. 1. Reference numeral 13 denotes a key-shaped projection of the hollow tube. FIG. 4A shows a combination of the sound insulation element, the hollow tube and the earphone. The projection is provided on the circumference of the hollow tube such that the sound insulation is prevented from being easily fallen out under a state where the sound insulation element is put on the hollow tube. The projection is key-shaped so that it can be easily inserted and prevented from being easily fallen out. A positional relationship between the hollow tube and the sound insulation element is important in obtaining a stable sound insulation characteristic. FIG. 4B is an enlarged view of the key-shaped projection of the hollow tube. The key-shape projection may be positioned in any places in the hollow tube. In addition, it is necessary and sufficient if only the key-shaped projection is shaped to be prevented from being separated.

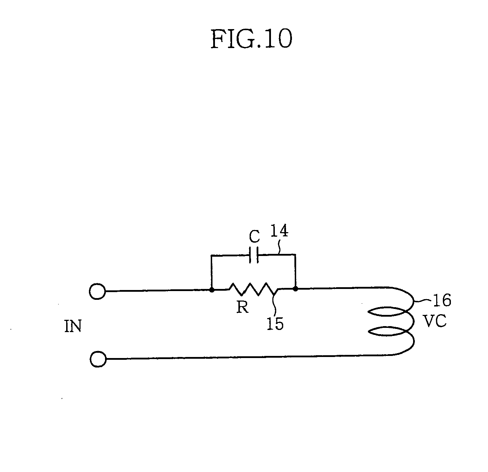

FIG. 10 shows a high sound range attenuation compensation circuit of the earphone according to one embodiment of the present invention.

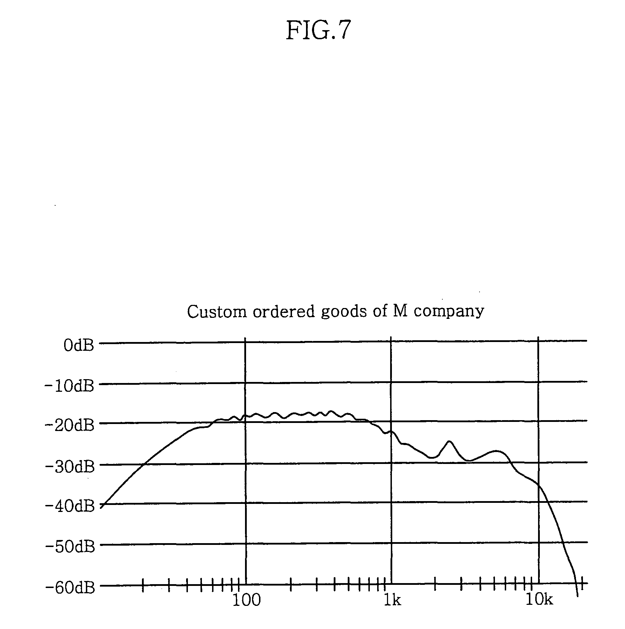

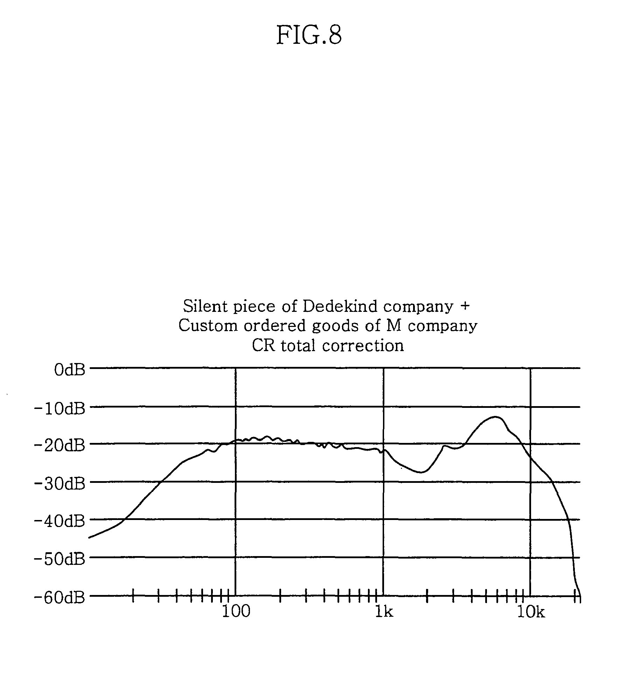

An embodiment of changing a regeneration characteristic of FIG. 7 to a regeneration characteristic of FIG. 8 is given by way of example as follows. In FIG. 10, VC represents an earphone voice coil, R represents a resistor, C represents a capacitor, and IN represents an earphone input terminal. A signal of a low sound range is attenuated by the resistor and a signal of a high sound range passes through the capacitor, thereby obtaining a low sound-limited and high sound-enhanced characteristic. In this embodiment, assuming that impedance of the voice coil is 16 Ω, the resistor has resistance of 32 Ω and the capacitor has capacitance of 4.7 7 µF, a -10dB attenuation is achieved in a middle and low sound range, thereby obtaining a high sound range-enhanced characteristic providing a gain of octave of +6dB from a frequency of about 3000Hz. The -10dB attenuation in the middle and low sound range has no practical problem since a general player outputs a large volume required to listen music under noisy environments.

FIG. 5 shows an {earphone sound insulation characteristic} of an available goods attached with a {highest performance sound insulation piece}.

In the figure, a dashed line represents a level of sound when a vertical axis is at a 0dB position and no earphone is mounted. Although the earphone sound insulation characteristic seems to show a sound insulation effect below a frequency of 80Hz, which is because a source of simulation environmental noise does not regenerate a sound of less than 80Hz, there is no attenuation of a range of less than 80Hz in an actual sound insulation characteristic. A small change in a frequency range of less than 30Hz is attributed to natural environmental noise in which a measurement instrument is placed.

A frequency range of less than 400Hz shows a right and down falling attenuation characteristic from {6dB to 7dB}. The attenuation slowly increases near a frequency range of more than 400Hz and an attenuation of about 35dB is shown in a frequency range of more than 1500Hz. This sound insulation characteristic is sufficiently useful under relatively light noise environments but insufficient under noisy (for example, 80dB) environments such as in a subway. FIG. 5 shows a sample having excellent sound insulation performance among available earphones, however, most of cheap ones have a sound insulation characteristic as poor as not to be put in practical use.

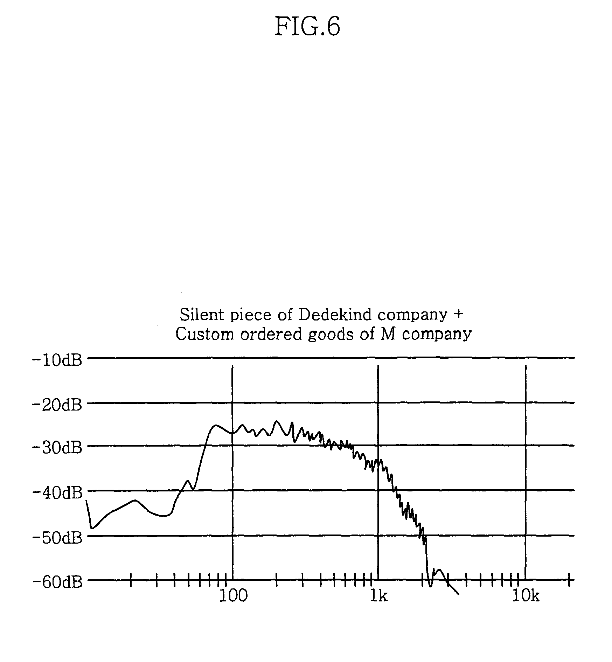

FIG. 6 shows a sound insulation characteristic when a composite sound insulation element is mounted on an earphone having the characteristic shown in FIG. 5.

In FIG. 6, a dashed line represents a level of sound when a vertical axis is at a 0dB position and no earphone is mounted. Although the earphone sound insulation characteristic seems to show a sound insulation effect below a frequency of 80Hz, which is because a source of simulation environmental noise does not regenerate a sound of less than 80Hz, there is no attenuation of a range of less than 80Hz in an actual sound insulation characteristic. A small change in a frequency range of less than 30Hz is attributed to natural environmental noise in which a measurement instrument is placed.

A frequency range of less than 400Hz shows a right and down falling attenuation characteristic from {17dB to 18dB}. The attenuation slowly increases near a frequency range of more than 400Hz and, unlike FIG. 6, noticeably increases even in a frequency range of more than 1500Hz. This sound insulation characteristic is sufficiently useful even under heavy noise environments such as in a subway and a jet liner. In reality, field experiments showed improved sound insulation performance of 10dB to 20dB as compared to conventional types.

FIG. 7 shows a regeneration characteristic of an {earphone equipped with the {composite sound insulation element} of the present invention.

Since the sound insulation element blocks a regeneration sound as well, the regeneration characteristic is attenuated from above 500Hz. An unequal characteristic in a range of from 1000Hz to 10000Hz is attributed to an inherent property of a measurement system. An actual attenuation characteristic is attenuated with an {inclination of octave of about 6dB}. Such an attenuation characteristic provides insufficient quality of high sound.

FIG. 8 shows a regeneration characteristic when an {example of the CR type high sound range attenuation compensation circuit} is interposed {between the voice coil and an earphone plug} of the {earphone on which the composite sound insulation element of the present invention is mounted}. Specifically, this figure shows a regeneration characteristic when a {volume of a signal source is preset to be large} such that {regeneration performance in a range of from 100Hz to 500Hz} is equal to that shown in FIG. 6. Considering an error of a measurement system, it is shown that the CR type high sound range attenuation compensation circuit maintains an ideal regeneration characteristic up to near 10kHz.

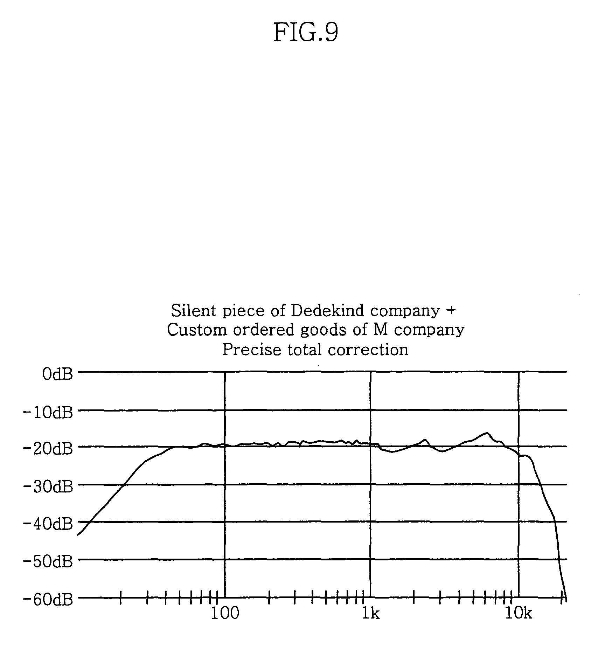

FIG. 9 shows a regeneration characteristic when an {example of a precise high sound range attenuation compensation circuit} is interposed {between the voice coil and the earphone plug} of the {earphone on which the composite sound insulation element of the present invention is mounted}. In this case, since the compensation circuit uses a digital signal processor, it is shown that substantially the same fine correction is made in a range of from 40Hz to 10000Hz as compared to that of FIG. 8.

As described above, by employing a {structure where the {sound insulation element mounted on the earphone blocks a portion of the external auditory canal}, it is possible to implement high performance noise reduction {even in a rear open type earphone}.

On the other hand, from the fact that a {close type earphone which seldom obtains good low sound regeneration performance} has a high sound range attenuation compensation characteristic similar to that of the present invention, the spirit of the present invention can be applied to a close type earphone having a simple structure.

Thus, it is possible to simply reduce environmental noise and obtain high quality of sound having little distortion and electronic circuit noise at a low cost and with stability using {conventional manufacture technology and equipment}.

In addition, since the {sound insulation} is inexpensive, it may be easily replaced with a new one {even if it is dirtied, damaged or lost}.

In addition, there is no need of a {difficult signal processing technique} that a {point of compromise of contradictory problems has to be considered} in connection with {stability of a closed loop including a complicated filter} and a {problem of noticeable deterioration of controllability due to a nonlinear characteristic of a system}.

### [Embodiment 1]

An environmental noise reduction type earphone which is commercialized separately.

A sound insulation element with various exchangeable {designs and forms}.

A sound insulation element for earphone.

An earphone mounted with a sound insulation element.

An earphone with a removable sound insulation element.

A sound insulation element which can be mounted on an earphone.

An earphone for mobile phone.

An earphone for potable player.

An earphone for aircraft seat.

The earphone of the present invention has an extremely wide range of application since it is practical, inexpensive, simple to be manufactured, easy to be used, and provides high reduction performance and high regeneration quality. Accordingly, the present invention can be applied to almost all apparatuses with which user are likely to hear sound under noisy environments.

## Claims

1. An earphone in which:
an inner earphone is defined as an earphone,
{environmental noise reaching an auditory sense via an earphone structure} is defined as entrance noise A,
{environmental noise reaching the auditory sense via a gap formed between the earphone and a {wall of an {external ear}} is defined as entrance noise B,
{sound reaching the auditory sense} of regeneration sound of the earphone is defines as entrance regeneration sound,
{deformable sound insulation porous plastic} to {fill a space formed near an entrance of the earphone and an external auditory canal} is defined as a sound insulation element,
a hole {through which a {hollow tube to be described later is inserted in the sound insulation element}} is defined as a hollow tube insertion hole,
the hollow tube insertion hole has a cup shape with one side closed and the other side opened,
an {opened side} of a {hole of the sound insulation element} is defined as an earphone mounting section,
a {closed side} of the {hole of the sound insulation element} is defined as a sound insulation section,
a {side along an axis} of the {hole of the sound insulation element} is defined as a sound insulation side,
an {empty tube having both} of a {function of being mounting on radiating holes of sound of the earphone} and a {function of combining the earphone and the sound insulation element by being inserted in the hole of the sound insulation element} is defined as a hollow tube;
the hollow tube has:
a {function as an aggregate of a {deformable soft sound insulation element}};
a {function of compressing {the sound insulation side and the sound insulation section} of the {sound insulation element filling a gap} between {the wall of the external ear} and the {hollow tube};
a {{function of attenuating the entrance noise B} by compressing a portion contacting the sound insulation side of the sound insulation element, i.e., the {wall of the external ear near the entrance of the external auditory canal}}; and
a function of attenuating {the entrance noise A, the entrance noise B, and the entrance regeneration sound} altogether by compressing the sound insulation section of the sound insulation element, i.e., {the vicinity of a space at the entrance of the external ear},
a combination of the sound insulation element and the hollow tube is defined as a composite sound insulation element,
an {{attenuation characteristic} or {dependency of the attenuation characteristic on frequency}} of a {{transmission characteristic of the entrance regeneration sound} of the {sound insulation element or composite sound insulation element}} is defined as an entrance regeneration sound attenuation characteristic,
a function of compensating the entrance regeneration sound attenuation characteristic is defined as a high sound range attenuation compensation function,
an earphone having a {regeneration characteristic of the high sound range attenuation compensation function} is defined as a high sound range attenuation compensation type earphone,
an {electronic circuit or electrical circuit} having a characteristic of the high sound range attenuation compensation function is defined as a high sound range attenuation compensation circuit,
an earphone {mounted with the composite sound insulation element} is defined as a first characteristic,
an {earphone having the high sound range attenuation compensation circuit at any place} between the {voice coil of the earphone} and the {earphone plug} is defined as a second characteristic, and
a high sound range attenuation compensation type earphone {mounted with the composite sound insulation element} is defined as a third characteristic,
wherein the earphone is the {earphone having the first and second characteristics} or the {earphone having the third characteristic}.

2. An earphone comprising the composite sound insulation element according to claim 1.

3. An earphone comprising the composite sound insulation element according to claim 1, wherein a sound insulation element separation prevention structure formed in any place on the circumference of the hollow tube is defined as a fourth characteristic.

4. The earphone according to claim 1, wherein the high sound range attenuation compensation type earphone has a ninth characteristic that high sound range regeneration sensitivity of the earphone is set to be higher than middle and low sound range regeneration sensitivity of the earphone by any one of:
a method of lowering low sound range sensitivity by decreasing a diameter of a diaphragm of the earphone;
a method of lowering low sound range sensitivity by increasing a strength of the diaphragm of the earphone;
a method of lowering low sound range sensitivity by decreasing a rear volume with a boundary of the diaphragm of the earphone; and
a method of lowering low sound range sensitivity by forming through holes in a rear portion and a front portion with a boundary of the diaphragm of the earphone.

5. An earphone in which:
{the high sound range attenuation compensation circuit according to claim 1} including a {parallel connection of a resistor and a capacitor} is defined as a CR type high sound attenuation compensation circuit,
insertion of the CR type high sound range attenuation compensation circuit between a {terminal of a voice coil of the earphone} and a {terminal of a lead drawn out of the earphone} is defined as a fifth characteristic,
insertion of the CR type high sound range attenuation compensation circuit in any place of the lead of the earphone is defined as a sixth characteristic, and
insertion of the CR type high sound range attenuation compensation circuit between the {lead of the earphone} and an {insertion plug of the earphone} is defined as a seventh characteristic,
wherein the earphone has the {fifth, sixth, or seventh} characteristic.

6. A {relay cord or a relay plug jack} comprising one terminal having an earphone plug and the other terminal having an earphone jack,
wherein insertion of a CR type high sound range attenuation compensation circuit between the earphone plug and the earphone jack is defined as an eighth characteristic.

7. An earphone comprising:
an earphone body which includes a voice coil and outputs a regeneration sound through radiating holes;
a sound insulation element which is made of deformable sound insulating porous plastic, covers the radiating holes of the earphone body, and fills a space formed between the earphone body and an entrance of an external auditory canal; and
a hollow tube which is mounted on the radiating holes of the earphone body and forms an aggregate such that the sound insulation element is combined to the earphone body.

8. The earphone according to claim 7, wherein the hollow tube includes an engagement projection formed on the circumference of the hollow tube and by which the sound insulation element is fixed to the hollow tube.

9. The earphone according to claim 7, wherein the earphone includes a high sound range attenuation correction circuit including a capacitor and a resistor connected in parallel between the voice coil of the earphone and a plug of the earphone.
